# EUROPEAN PATENT APPLICATION

(11) **EP 2 308 883 A1**
(43) Date of publication of application: **13.04.2011**
(21) Application number: 09172257.9
(22) Date of filing: 05.10.2009
(51) Int. Cl.: C07D 495/04, C07D 495/14, A61K 31/4365, A61K 31/519, A61P 33/06

(54) **New derivatives of thieno[2,3-b]pyridine and 5,6,7,8 tetrahydrothieno[2,3 b]quinoline in particular useful in the treatment of malaria**

(71) Applicant: CENTRE NATIONAL DE LA RECHERCHE SCIENTIFIQUE, 75794 Paris Cedex (FR)
(72) Inventor: MEIJER, Laurent, 29680, ROSCOFF (FR); KUNICK, Conrad, 21149, HAMBURG (DE); BRANDT, Wiebke, 38114, BRAUNSCHWEIG (DE); DOERIG, Christian, 1110, MORGES (CH)
(74) Representative: Le Coupanec, Pascale A.M.P.

(57) **Abstract**

The present invention is related to compounds of formula (I) 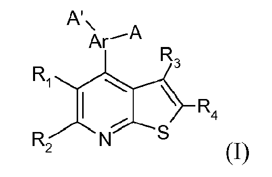 in particular useful for the treatment of malaria, and to pharmaceutical compositions containing them.

## Description

The present invention is in the field of treatment of malaria.

The present invention more precisely deals with new derivatives of thieno[2,3-*b*]pyridine and 5,6,7,8-tetrahydrothieno[2,3-*b*]quinoline in particular useful for the treatment of malaria. It also relates to the corresponding pharmaceutical compositions containing them.

Malaria is responsible each year for 250-300 million clinical cases and for 1 to 3 millions deaths. *Plasmodium falciparum* is the parasite of malaria that causes most of said deaths but other species such *as P. vivax, P. malariae, P. ovale, P. knowlesi* contribute to malaria in various areas. *P. falciparum* is the most dangerous of these infections as *P. falciparum* (or malignant) malaria has the highest rates of complications and mortality.

The active agents for the treatment of malaria that are available up to the end of the last millenium come out of the following substance groups:
- 4-Aminochinoline (Chloroquine und Amodiaquine)
- Arylaminoalcohol (Quinine, Mefloquine, Lumefantrine)
- 8-Aminochinoline (Primaquine)
- Antifolate (Sulfadoxine, Pyrimethamine, Proguanil und Chlorproguanil)
- Antibiotic (Tetracycline)
- Hydroxynaphthochinone (Atovaquon).

Through mutations in certain genes of the malaria pathogen *Plasmodium falciparum,* resistant strain against almost all active agents named above have developed in the meantime. Artemisin and some derivatives thereof (Artemether and Artesunat) are now proposed most of the time in combination therapy in most of Africa countries. However there does not exist a real alternative to such treatment in particular in case where a resistance to Artemisin derivatives would appear.

Therefore, there still exists a need to develop new chemical structures with new biological pathways due to worldwide increasing resistance of *Plasmodiumfalciparum.*

New active agents were developed in many substance groups as for example the Pyronaridin and the Piperaquin in the group of the 4-Aminochinoline. Both substances stand as a combination preparation (Pyronaridin/Artesunat and Piperaquin/Dihydroartemisinin) shortly before their allowance.

Other substances inhibitors of the Cytochrom bcl-complexes of the respiratory chain are under development and possess a similar bilogical mechanism such as the Atovaquon. A representative of these 4-Pyridone are going to start a clinical development.

Among such other substances the following may further be cited: Fosmidomycin, bisammonium derivatives like T3 and parfuramidin.

Document Droucheau, E et al.: Plasmodium Falciparum glycogen synthase kinase-3: molecular model, expression, intracellular localisation and selective inhibitors Biochimica et Biophysica Acta 1697 2004, 181-196 states that Glycogen synthase kinase-3 (GSK-3) represents a potential screening target for the identification of new antimalaria compounds. It was indeed previouly stated that protein kinases could constitute attractive candidates as potentiel targets for inhibiting a physiological process essential to the parasite. Even if said document suggests that PfGSK-3 selective compounds might be identified to develop new antimalaria drugs, it is mute on potent particular chemical structures fulfilling said objective, except indirubins; which are not specific.

Moreover, the document Sharanin, Y. A., Krivokolysko, S. G., Dyachenko, V. D.: Cyclization reactions of nitriles LIV. Synthesis and properties of 6-amino-4-aryl-3,5-dicyanopyridin-2(1H)-ones, the corresponding thiones, the pyridylidenemalononitriles and their hydrogenated analogs. Russ. J. Org. Chem. 1994, 30, 620-626 discloses derivatives of thieno[2,3-*b*]pyridine with no associated biological activity.

The inventors have surprisingly found that the compounds of formula (I) as defined hereinafter inhibit the enzymatic activity of *Pf*GSK-3. They can therefore be of particular interest in the treatment of malaria.

As a first object, the present invention deals with a compound of formula (I) 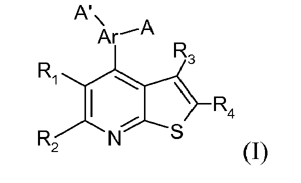 wherein
**R₁** represents a -CN group and **R₂** represents a -NH₂ group or alternatively **R₁** and **R₂** together form a cyclcohexyl ring fused with the thieno[2,3-*b*]pyridine group,
**R₃** represents a -NH₂ group, or a NHCOR₇ group, wherein R₇ is a (C₁-C₄) alkyl group,
**R₄** represents a -CN group or a formula (A) 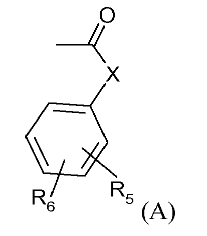
wherein
**R₅** and **R₆** independently represent a hydrogen atom, a halogen atom, a trifluoromethyl group, a -CN group, a (C₁-C₄)alkoxy or a (C₁-C₄)alkyl group, and
**X** represents a bond or a -NH- group, wherein alternatively R₃ and R₄ together form a ring of formula (B), fused to the thieno[2,3-*b*]pyridine 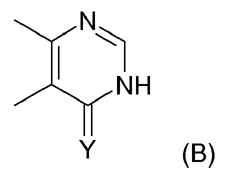
wherein
Y is an oxygen or a sulphur atom,
**Ar** represents a phenyl group, or a heteroaryl group chosen among thienyl, indolyl and furyl,groups,
**A** and **A'** independently represent a hydrogen atom, a (C₁-C₄)alkoxy group or a halogen atom,
wherein when **Ar** is a phenyl group, at least one of A or A' is different form a hydrogen atom and is in the ortho position, and
wherein (C₁-C₄)alkoxy group and (C₁-C₄)alkyl group may potentially independently be substituted by one group chosen among a -NH₂ group and a -COOH group,
with the exclusion of the following four compounds of formula (I) :
- (i) and (ii) wherein **Ar** is a phenyl group, **R₁** is a -CN group, **R₂** and **R₃** are each a -NH₂ group, **A** is a o-I or a o-Cl and **A'** is a hydrogen atom, **R₄** represents a formula (A) wherein **X** is a bond, **R₅** is a hydrogen atom and **R₆** is a p-Cl, and
- (iii) and (iv) wherein **Ar** is a furyl group, **R₁** is a -CN group, **R₂** and **R₃** are each a -NH₂ group, **A** and **A'** are hydrogen atom, **R₄** represents a formula (A) wherein X is a bond, **R₅** is a hydrogen atom and **R₆** is a p-Cl or a hydrogen atom,
or anyone of their salts with pharmaceutically acceptable acids and mixtures thereof,

According to a particular embodiment, the compound of formula (I) is as above-defined and more particularly, independently or considered together:
- R₃ represents a -NH₂ group, and/or
- Ar represents a phenyl group, a thienyl group or an indolyl group; and more particularly a phenyl group, a 2-thienyl group or an indol-3-yl group.

According to another particular embodiment, when A, A', R₅ or R₆ represent a (C₁-C₄)alkoxy group or a (C₁-C₄) alkyl group, said groups are unsubstituted.

As a further object, the present invention deals with 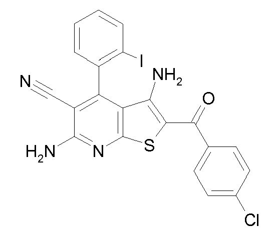 or 3,6-diamino-2-(4-chlorobenzoyl)-4-(2-iodophenyl)thieno[2,3-*b*]pyridine-5-carbonitrile,
as a medicament, in particular useful to treat malaria.

Said particular compound is present in the table as compound 1.

The present invention further relates to 3,6-diamino-2-(4-chlorobenzoyl)-4-(2-chlorophenyl)thieno[2,3-*b*]pyridine-5-carbonitrile; 3,6-diamino-2-(4-chlorobenzoyl)-4-(2-furyl)thieno[2,3-*b*]pyridine-5-carbonitrile or 3,6-diamino-2-benzoyl-4-(2-furyl)thieno[2,3-*b*]pyridine-5-carbonitrile as a medicament, in particular useful to treat malaria.

The present invention further provides a compound of formula (Ia) 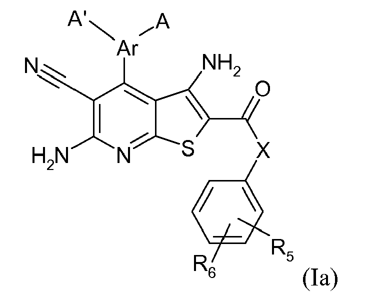 wherein
**Ar** represents a phenyl group or a 2-thienyl group,
**A** and **A'** independently represent a hydrogen atom, a (C₁-C₄)alkoxy group or a halogen atom,
**X** represents a bond or a -NH- group,
**R₅** and **R₆** independently represent a hydrogen atom, a halogen atom, a (C₁₋C₄)alkoxy group, a (C₁-C₄)alkyl group, a trifluoromethyl group, or a -CN group, and
wherein when **Ar** is a phenyl group, at least one of A or A' is different form a hydrogen atom and is in the ortho position, and
wherein (C₁-C₄)alkoxy group and (C₁-C₄)alkyl group may potentially independently be substituted by one group chosen among a -NH₂ group and a -COOH group,
with the exclusion of the following four compounds of formula (I) :
- (i) and (ii) wherein **Ar** is a phenyl group, **R₁** is a -CN group, **R₂** and **R₃** are each a -NH₂ group, **A** is a o-I or a o-Cl and **A'** is a hydrogen atom, **R₄** represents a formula (A) wherein **X** is a bond, **R₅** is a hydrogen atom and **R₆** is a p-Cl, and
- (iii) and (iv) wherein **Ar** is a furyl group, **R₁** is a -CN group, **R₂** and **R₃** are each a -NH₂ group, **A** and **A'** are hydrogen atom, **R₄** represents a formula (A) wherein X is a bond, **R₅** is a hydrogen atom and **R₆** is a p-Cl or a hydrogen atom.

According to a particular embodiment, the compound of formula (Ia) is such as defined above with **X** being a bond.

The present invention moreover provides a compound of formula (I'a) 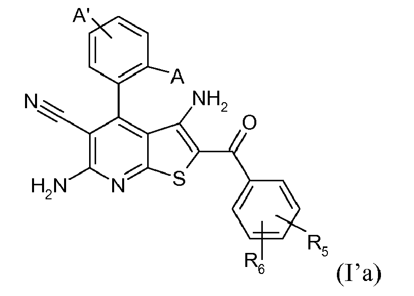 wherein
**A** represents a (C₁-C₄)alkoxy group or a halogen atom,
**A'** represents a hydrogen atom, a (C₁-C₄)alkoxy group or a halogen atom,
**R₅** and **R₆** independently represent a hydrogen atom, a halogen atom, a (C₁-C₄)alkoxy group, a (C₁-C₄)alkyl group, a trifluoromethyl group, a -CN group, and
wherein (C₁-C₄)alkoxy group and (C₁-C₄)alkyl group may potentially independently be substituted by one group chosen among a -NH₂ group and a -COOH group,
with the exclusion of the two compounds of formula (I) (i) and (ii) wherein **A** is a o-I or o-Cl and **A'** is a hydrogen atom, **R₅** is a hydrogen atom and **R₆ is** a p-Cl.

The present invention further relates to a compound of formula (Ib) 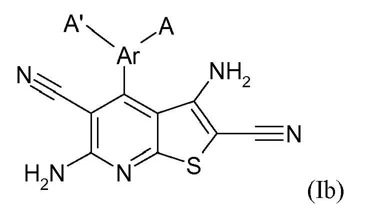 wherein
**Ar** represents a phenyl group, a 2-thienyl group or an indol-3-yl group,
**A** and **A'** independently represent a hydrogen atom, a (C₁-C₄)alkoxy group or a halogen atom,
wherein (C₁-C₄)alkoxy group and (C₁-C₄)alkyl group may potentially independently be substituted by one group chosen among a -NH₂ group and a -COOH group, and
wherein when **Ar** is a phenyl group, at least one of A or A' is different from a hydrogen atom and is in the ortho position.

The present invention further relates to a compound of formula (I'b) 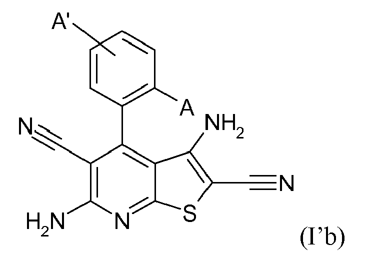 wherein
**A** represents a (C₁-C₄)alkoxy group or a halogen atom,
**A'** represents a hydrogen atom, a (C₁-C₄)alkoxy group or a halogen atom, and
wherein (C₁-C₄)alkoxy group and (C₁-C₄)alkyl group may potentially independently be substituted by one group chosen among a -NH₂ group and a -COOH group.

The present invention moreover provides a compound of formula (Ic) 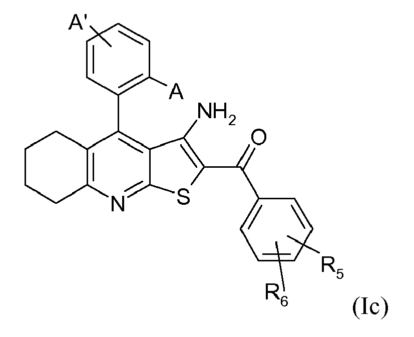 wherein
**A** represents a (C₁-C₄)alkoxy group or a halogen atom,
**A'** represents a hydrogen atom, a (C₁-C₄)alkoxy group or a halogen atom,
**R₅** and **R₆** independently represent a hydrogen atom, a halogen atom, a (C₁-C₄)alkoxy group, a (C₁-C₄)alkyl group, a trifluoromethyl group, a -CN group, and
wherein (C₁-C₄)alkoxy group and (C₁-C₄)alkyl group may potentially independently be substituted by one group chosen among a -NH₂ group and a -COOH group.

Moreover, the present invention provides a compound of formula (1d) 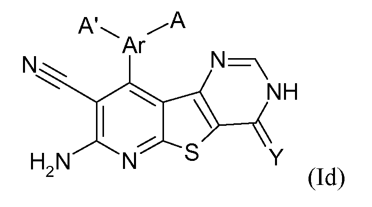 wherein
**Ar** represents a phenyl group or a 2-thienyl group,
**A** and **A'** independently represent a hydrogen atom, a (C₁-C₄)alkoxy group or a halogen atom, and
**Y** represents a sulphur or an oxygen atom,
wherein when **Ar** is a phenyl group, at least one of A or A' is different from a hydrogen atom and is in the orthoposition, and
wherein (C₁-C₄)alkoxy group and (C₁-C₄)alkyl group may potentially independently be substituted by one group chosen among a -NH₂ group and a -COOH group.

In the framework of the present invention, the substitution groups have the following meanings:
- the term "alkoxy" refers to a saturated straight or branched-chain hydrocarbon radical attached to an oxygen atom. Included within the scope of this term are such moieties as methoxy, ethoxy, propoxy, isopropoxy, n-butoxy, isobutoxy, sec-butoxy, *tert*-butoxy and the like,
- the term "alkyl" as used herein refers to a straight or branched-chain hydrocarbon radical and cyclized manifestations thereof unless otherwise indicated. Included within the scope of this term are such moieties as methyl, ethyl, n-propyl, isopropyl cyclopropyl, n-butyl, isobutyl, *sec*-butyl, *tert*-butyl, and the like,
- the term "halogen" refers to a fluorine, a chlorine, a bromine or an iodine atom, and
- o- designates the ortho position, m- designates the meta position and p- designates the para position,

According to a particular embodiment, the present invention moreover provides the use of a compound of formula (I), (Ia), (I'a), (Ib), (I'b), (Ic) or (Id) as defined above for the manufacture of a pharmaceutical composition intended for the treatment of malaria.

According to another aspect, the present invention further relates to a compound of formula (I), (Ia), (I'a), (Ib), (I'b), (Ic) or (Id) as defined above useful for the treatment of malaria.

Among compounds of formula (Ia), the following compounds, including their salts, may be particularly cited. The numbers given after the chemical name correspond to the numbers given in the table below:
- 3,6-diamino-2-benzoyl-4-(2-methoxyphenyl)thieno[2,3-*b*]pyridine-5-carbonitrile (2)
- 3,6-diamino-2-(4-chlorobenzoyl)-4-(2-methoxyphenyl)thieno[2,3-*b*]pyridine-5-carbonitrile (3)
- 3,6-diamino-2-(4-methoxybenzoyl)-4-(2-methoxyphenyl)thieno[2,3-*b*]pyridine-5-carbonitrile (4)
- 3,6-diamino-4-(2-methoxyphenyl)-2-(4-methylbenzoyl)thieno[2,3-*b*]pyridine-5-carbonitrile (5)
- 3,6-diamino-4-(2-methoxyphenyl)-2-[4-(trifluoromethyl)benzoyl]thieno[2,3-*b*]pyridine-5-carbonitrile (6)
- 3,6-diamino-2-(3-chlorobenzoyl)-4-(2-methoxyphenyl)thieno[2,3-*b*]pyridine-5-carbonitrile (7)
- 3,6-diamino-2-(2-chlorobenzoyl)-4-(2-methoxyphenyl)thieno[2,3-*b*]pyridine-5-carbonitrile (8)
- 3,6-diamino-2-(4-cyanobenzoyl)-4-(2-methoxyphenyl)thieno[2,3-*b*]pyridine-5-carbonitrile (9)
- 3,6-diamino-2-benzoyl-4-(2-iodophenyl)thieno[2,3-*b*]pyridine-5-carbonitrile (10)
- 3,6-diamino-4-(2-iodophenyl)-2-(4-methoxybenzoyl)thieno[2,3-*b*]pyridine-5-carbonitrile (11)
- 3,6-diamino-4-(2-iodophenyl)-2-(4-methylbenzoyl)thieno[2,3-*b*]pyridine-5-carbonitrile (12)
- 3,6-diamino-4-(2-iodophenyl)-2-[4-(trifluoromethyl)benzoyl]thieno[2,3-b]pyridine-5-carbonitrile (13)
- 3,6-diamino-2-(3-chlorobenzoyl)-4-(2-iodophenyl)thieno[2,3-*b*]pyridine-5-carbonitrile (14)
- 3,6-diamino-2-(2-chlorobenzoyl)-4-(2-iodophenyl)thieno[2,3-*b*]pyridine-5-carbonitrile (15)
- 3,6-diamino-2-(3,4-dichlorobenzoyl)-4-(2-iodophenyl)thieno[2,3-*b*]pyridine-5-carbonitrile (16)
- 3,6-diamino-2-(4-chlorobenzoyl)-4-(2-ethoxyphenyl)thieno[2,3-*b*]pyridine-5-carbonitrile (17)
- 3,6-diamino-2-benzoyl-4-(2-bromophenyl)thieno[2,3-*b*]pyridine-5-carbonitrile (18)
- 3,6-diamino-4-(2-bromophenyl)-2-(4-chlorobenzoyl)thieno[2,3-*b*]pyridine-5-carbonitrile (19)
- 3,6-diamino-4-(2-bromophenyl)-2-(3-chlorobenzoyl)thieno[2,3-*b*]pyridine-5-carbonitrile (20)
- 3,6-diamino-4-(2-bromophenyl)-2-(3,4-dichlorobenzoyl)thieno[2,3-b]pyridine-5-carbonitrile (21)
- 3,6-diamino-2-(3-chlorobenzoyl)-4-(2-chlorophenyl)thieno[2,3-*b*]pyridine-5-carbonitrile (22)
- 3,6-diamino-4-(2-chlorophenyl)-2-(3,4-dichlorobenzoyl)thieno[2,3-b]pyridine-5-carbonitrile (23)
- 3,6-diamino-4-(2-chlorophenyl)-2-(3-methoxybenzoyl)thieno[2,3-*b*]pyridine-5-carbonitrile (24)
- 3,6-diamino-4-(2-chlorophenyl)-2-[3-(trifluoromethyl)benzoyl]thieno[2,3-b]pyridine-5-carbonitrile (25)
- 3,6-diamino-4-(2-chlorophenyl)-2-(3-cyanobenzoyl)thieno[2,3-*b*]pyridine-5-carbonitrile (26)
- 3,6-diamino-2-(3-bromobenzoyl)-4-(2-chlorophenyl)thieno[2,3-*b*]pyridine-5-carbonitrile (27)
- 3,6-diamino-4-(2-chlorophenyl)-2-(3-fluorobenzoyl)thieno[2,3-*b*]pyridine-5-carbonitrile (28)
- 3,6-diamino-2-(3-chlorobenzoyl)-4-(2-fluorophenyl)thieno[2,3-*b*]pyridine-5-carbonitrile (29)
- 3,6-diamino-2-(3-chlorobenzoyl)-4-(2,6-dichlorophenyl)thieno[2,3-*b*]pyridine-5-carbonitrile (30)
- 3,6-diamino-2-(3-chlorobenzoyl)-4-(2-thienyl)thieno[2,3-*b*]pyridine-5-carbonitrile (31)
- 3,6-diamino-*N*-(4-chlorophenyl)-5-cyano-4-(2-iodophenyl)thieno[2,3-*b*]pyridine-2-carboxamide (32)
- 3,6-diamino-4-(2-bromophenyl)-*N*-(4-chlorophenyl)-5-cyanothieno[2,3-*b*]pyridine-2-carboxamide (33)
- 3,6-diamino-4-(2-chlorophenyl)-*N*-(4-chlorophenyl)-5-cyanothieno[2,3-*b*]pyridine-2-carboxamide (34)

Among compounds of formula (Ib), the following compounds, including their salts, may be particularly cited. The numbers given after the chemical name correspond to the numbers given in the table below:
- 3,6-diamino-4-(2-methoxyphenyl)thieno[2,3-*b*]pyridine-2,5-dicarbonitrile (35)
- 3,6-diamino-4-(2-iodophenyl)thieno[2,3-*b*]pyridine-2,5-dicarbonitrile (36)
- 3,6-diamino-4-(2-bromophenyl)thieno[2,3-*b*]pyridine-2,5-dicarbonitrile (37)
- 3,6-diamino-4-(2-chlorophenyl)thieno[2,3-*b*]pyridine-2,5-dicarbonitrile (38)
- 3,6-diamino-4-(2-ethoxyphenyl)thieno[2,3-*b*]pyridine-2,5-dicarbonitrile (39)
- 3,6-diamino-4-(2-methylphenyl)thieno[2,3-*b*]pyridine-2,5-dicarbonitrile (40)
- 3,6-diamino-4-(2-thienyl)thieno[2,3-*b*]pyridine-2,5-dicarbonitrile (41)
- 3,6-diamino-4-(1*H*-indol-3-yl)thieno[2,3-*b*]pyridine-2,5-dicarbonitrile (42)

Among compounds of formula (Ic), the following compounds, including their salts, may be particularly cited. The numbers given after the chemical name correspond to the numbers given in the table below:
- [3-amino-4-(2-iodophenyl)-5,6,7,8-tetrahydrothieno[2,3-*b*]quinoline-2-yl](phenyl)methanone (43)
- [3-amino-4-(2-iodophenyl)-5,6,7,8-tetrahydrothieno[2,3-*b*]quinoline-2-yl](4-chlorophenyl)methanone (44)
- [3-amino-4-(2-bromophenyl)-5,6,7,8-tetrahydrothieno[2,3-*b*]quinoline-2-yl](phenyl)methanone (45)

Among compounds of formula (Id), the following compounds including their salts, may be particularly cited. The numbers given after the chemical name correspond to the numbers given in the table below:
7-amino-9-(2-chlorophenyl)-4-oxo-3,4-dihydropyrido[3',2':4,5]thieno[3,2-*d*]pyrimidine-8-carbonitrile (46)
7-amino-9-(2-chlorophenyl)-4-thioxo-3,4-dihydropyrido[3',2':4,5]thieno[3,2-*d*]pyrimidine-8-carbonitrile (47)

Some molecules represented by the general formula (I) or anyone of the formula (Ia), (I'a), (Ib), (I'b), (Ic) and (Id) are chiral because they exist in atropisomeric forms or possess one asymmetric carbon atom. They can exist in the form of pure enantiomers or as a mixture of enantiomers. Moreover, the compounds according to the invention exist in the form of free bases or of addition salts with pharmaceutically acceptable acids.

The pharmaceutically acceptable salts of the compounds of formulae (I), (Ia), (I'a), (Ib), (I'b), (Ic) and (Id) include the addition salts with pharmaceutically acceptable acids, such as inorganic acids, for example hydrochloric, hydrobromic, phosphoric or sulphuric acid, and organic acids, such as acetic, trifluoroacetic, propionic, oxalic, succinic, fumaric, malic, tartaric, citric, ascorbic, maleic, glutamic, benzoic, toluenesulphonic, methanesulphonic, stearic and lactic acid.

The compounds of formula (I), (Ia), (I'a), (Ib), (I'b), (Ic) and (Id) may also exist under the form of hydrates or solvates, i.e. under the form of associations or combinations with one or more water molecule(s) or with a solvent. Such hydrates and solvates also form part of the present invention.

The invention more particularly concerns the following compounds of formula (Ia) wherein X is a bond and Ar is a phenyl group: compounds (2), (7), (11), (12), (14), (18), (20), (21), (22), (23), (24), (25), (26), (27) and (30). Said compounds are more particularly cited as demonstrating particularly good anti-PfGSK-3 IC₅₀ values whereas they do not demonstrate good anti-mammalian GSK-3 IC₅₀ values.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

In accordance with a feature of the invention, the compounds of formula (Ia) and (Ib) are prepared according to scheme 1 below.

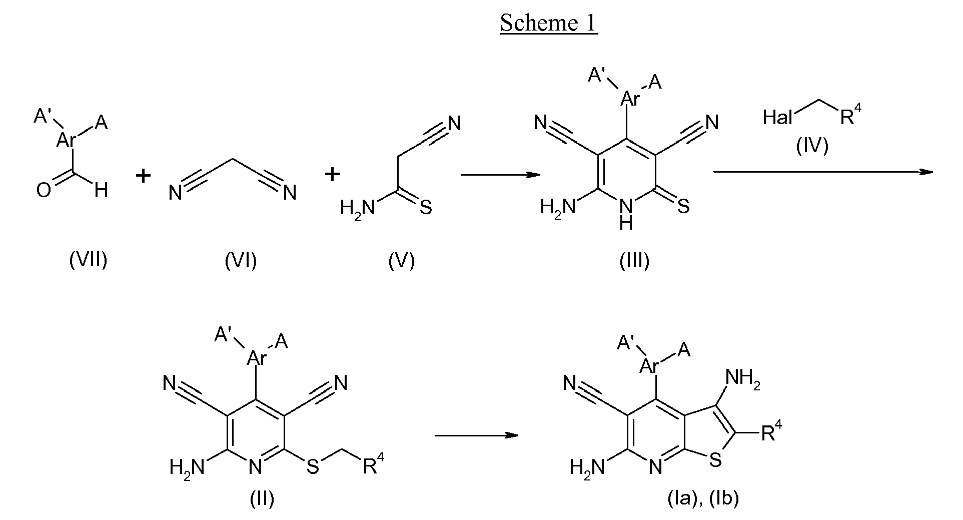

An aldehyde of formula (VII) in which Ar, A and A' are as defined above is reacted with malonodinitrile of formula (VI) and with cyanothiaocetamide of formula (V) in the presence of piperidine, in an organic solvent such as ethanol at the boiling point of the mixture, for example for a duration from 1 to 6 hours, and for example during 3 hours. A compound of formula (III) is obtained which is reacted with a compound of formula (IV) in which Hal is a halogen atom and R₄ is as defined above in the presence of KOH in dimethylformamide at 25°C to give a compound of formula (II). Said compound of formula (II) is reacted in the presence of KOH in dimethylformamide at 25°C to give a compound of formula (Ia), (Ib) or (Ic).

Compounds of formula (I'a) and (I'b) may also be prepared according to said scheme 1.

The compounds of formula (Id) wherein Y is an oxygen atom may be prepared starting from compounds of formula (Ia). The cyclization may be performed in acidic conditions for example in formic acid at reflux temperature for example during 0.5 to 10 hours.

The compounds of formula (Id) wherein Y is a sulphur atom may be prepared starting from compounds of formula (Id) wherein Y is an oxygen atom in the presence of Lawessons reagent in an organic solvent such as 1,4-dioxane, at reflux temperature for example during 0.1 to 3 hours .

The compounds of general formula (1c), may be prepared according to a further feature of the invention according to the following scheme 2.

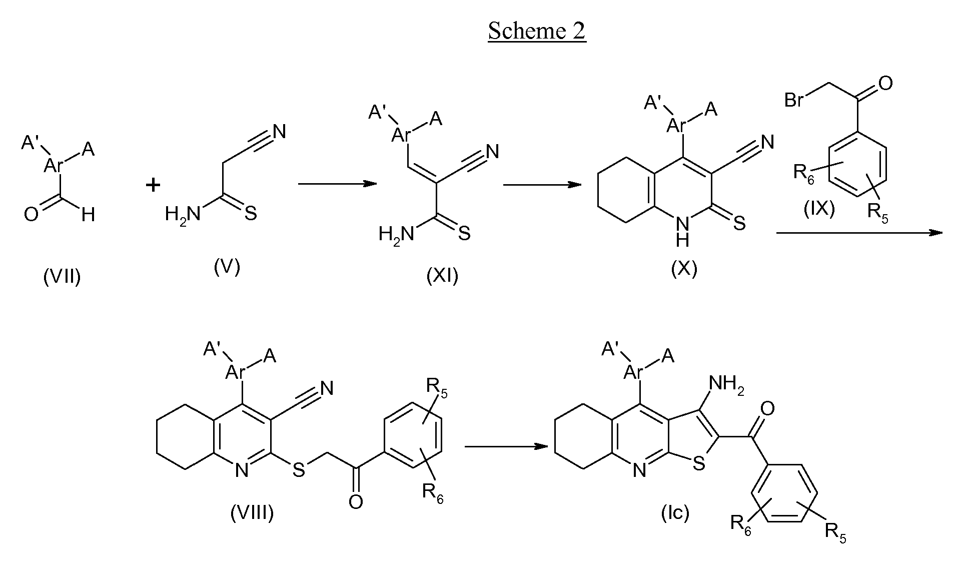

A compound of formula (VII) in which Ar, A and A' are as defined above are reacted with a compound of formula (V), in the presence of triethylamine, in an organic solvent such as ethanol at 40-50°C. A compound of formula (XI) is obtained, which is reacted with cyclohexanone in 1,4-dioxane in the presence of piperidine at reflux temperature to give a compound of formula (X). A compound of formula (X) is then reacted with a compound of formula (IX) in which R₅ and R₆ are as defined above in dimethylformamide in the presence of KOH at 25°C to give a compound of formula (VIII). Said compound of formula (VIII) is finally reacted in dimethylformamide in the presence of KOH at 25°C to give a compound of formula (Ic).

The starting compounds are commercially available or are described in the literature, or can be synthesized in accordance with methods which are described therein or which are known to the person skilled in the art, in particular, compounds of formula (V), (VI), (VII) or (XI).

Some intermediate compounds also form part of the present invention:
- Compound (II), wherein Ar, A, A' and R₄ are as defined above, with the exclusion of the following four compounds of formula (II):
- (I') and (ii') wherein Ar is a phenyl group, A is a o-I or a o-Cl and A' is a hydrogen atom, R4 represents a formula (A) wherein X is a bond, R₅ is a hydrogen atom and R₆ is a p-Cl, and
- (iii') and (iv') wherein Ar is a furyl group, A and A' are hydrogen atom, R₄ represents a formula (A) wherein X is a bond, R₅ is a hydrogen atom and R₆ is a p-Cl or a hydrogen atom, and
- Compound (VIII), wherein Ar, A, A', R₅ and R₆ are as defined above.

The following examples illustrate the preparation of some compounds according to the invention. The NMR spectra confirm the structures of the products obtained. The number given in brackets in the example titles correspond to those of the table which are given subsequently.

General procedure 1: synthesis of 6-amino-4-aryl-2-thioxo-1,2-dihydropyridine-3,5-dicarbonitriles of formula (III)

A mixture of the appropriate aldehyde of formula (VII) (1.00 mmol), malonodinitrile (VI) (1.00 mmol, 66.1 mg), cyanothioacetamide (V) (1.00 mmol, 100 mg) and piperidine (50 µl) in ethanol (4 ml) was refluxed for 3 h. Then the solvent was evaporated *in vacuo.* To the residue was added acetic acid (6 drops), water (10 ml) and dichloromethane (2 ml). The mixture was agitated for a short time. If a precipitate was not formed the mixture was stored at 2-5°C for some time. The precipitate was collected by suction and used for the following syntheses without further purification.

General procedure 2: syntheses of 3,6-diamino-2-aroyl-4-arylthieno[2,3-b]pyridine-5-carbonitriles **(compound of formula (Ia) wherein R₄ represents a group of formula (A) wherein X is a bond),** 3,6-diamino-4-aryl-N-(4-chlorophenyl)-5-cyanothieno[2,3-b]pyridine-2-carboxamides **(compound of formula (Ia) wherein R₄ represents a group of formula (A) wherein X is -NH-),** 3,6-diamino-4-arylthieno[2,3-b]pyridine-2,5-dicarbonitriles **(compound of formula (Ib))** and 3-amino-4-aryl-5,6,7,8-tetrahydrothieno[2,3-b]quinoline-2-yl](aryl)methanones **(compound of formula (Ic))** Said general procedure 2 is performed according to a method by Sharanin et al. as already cited above.

An appropriate 6-amino-4-aryl-2-thioxo-1,2-dihydropyridine-3,5-dicarbonitrile of formula (III) (0.400 mmol) or 4-aryl-2-thioxo-1,2,5,6,7,8-hexahydroquinoline-3-carbonitrile of formula (X) (0.400 mmol) was dissolved in DMF (0.5 ml). To the mixture was added a 10% aqueous solution of potassium hydroxide (224 µ1, 0.4 mmol). After 1 min an appropriate phenacylbromide, 2-chloro-N-(4-chlorophenyl)acetamide or chloroacetonitrile each of formula (IV) (0.400 mmol in each case) was added. After stirring at 25°C for 30 minutes 224 µl of 10% aqueous solution of potassium hydroxide was added to the mixture again. The reaction mixture was stirred at 25°C until the intermediates of formula (II) and of formula (VIII) respectively has reacted completely (reaction control by thin layer chromatography). After adding water (5 ml) the precipitate was collected by suction and washed with water and a little amount of ethanol 70% and petroleum ether. The precipitate was recrystallized from a solvent as stated.

### Example 1: 3,6-diamino-4-(2-iodophenyl)-2-(4-methoxybenzoyl)thieno[2,3-b]pyridine-5-carbonitrile (compound No. 11 of the Table)

According to general procedure 2 from 6-amino-4-(2-iodophenyl)-2-thioxo-1,2-dihydropyridine-3,5-dicarbonitrile (compound of formula (III)) (75.6 mg, 0.200 mmol), 10% aqueous solution of potassium hydroxide (112 µl twice, 0.400 mmol) and 2-bromo-1-(4-methoxyphenyl)ethanone (45.8 mg, 0.200 mmol). Reaction period: 40 min. Crystallization from ethanol afforded 45.2 mg (43%) of a yellow powder.

mp.: 316-317°C; IR (KBr): 3462 cm-1, 3305 cm-1 and 3199 cm-1 (NH), 2930 cm-1 (CH aliph), 2216 cm-1 (C≡N), 1620 cm-1 (C=O); 1H-NMR (DMSO-*d₆*, 400 MHz): δ (ppm) = 3.84 (s, 3 H, OCH₃), 6.50 (br s, 2 H, NH₂), 7.03-7.07 (m, 2 H, part of a AA'XX' system, ArH), 7.39 ("dt", 1 H, J = 7.6/1.5 Hz, ArH), 7.58 (dd, 1 H, J = 7.6/1.5 Hz, ArH), 7.67-7.72 (m, 5 H, NH₂ and ArH, overlapping), 8.14 (dd, 1 H, J = 8.1/1.0 Hz, ArH); 13C-NMR (DMSO-*d₆*, 100.6 MHz): δ (ppm) = 55.4 (OCH₃); 113.7 (2 C), 128.8, 129.2 (2 C), 129.3, 131.9, 139.5 (tert. C); 90.5, 97.3, 99.6, 112.4, 133.2, 138.2, 150.3, 155.0, 159.2, 161.4, 165.9, 186.9 (quart. C, 1 C not detected); C₂₂H₁₅IN₄O₂S (526.35); calcd C 50.20, H 2.87, N 10.64, S 6.09; found C 50.33, H 2.97, N 10.80, S 5.83; HPLC: 95.6% at 254 nm and 97.9% at 280 nm, t_{N} = 3.76 min, t_{M} = 1.01 min (ACN/H₂O; 55:45), λmax: 330 nm, 225 nm, 290 nm.

### Example 2: 3,6-diamino-4-(2-bromophenyl)-2-(3,4-dichlorobenzoyl)thieno[2,3-b]pyridne-5-carbonitrile (compound No.21 of the Table)

According to general procedure 2 from 6-amino-4-(2-bromophenyl)-2-thioxo-1,2-dihydropyridine-3,5-dicarbonitrile (compound of formula (III)) (133 mg, 0.401 mmol), 10% aqueous solution of potassium hydroxide (224 µl twice, 0.800 mmol) and 2-bromo-1-(3,4-dichlorophenyl)ethanone (107 mg, 0.400 mmol). Reaction period: 0.5 h. Crystallization from ethanol afforded 88.7 mg (43%) of a yellow powder.

mp.: 289-290°C; IR (KBr): 3475 cm⁻¹ and 3397 cm⁻¹ (NH), 2223 cm⁻¹ (C≡N); ¹H-NMR (DMSO-*d₆*, 600 MHz): δ (ppm) = 6.64 (br s, 2 H, NH₂), 7.60-7.70 (m, 4 H, ArH), 7.80 (d, 1 H, *J* = 8.3 Hz, ArH), 7.84 (br s, 2 H, NH₂, overlapped by d at 7.80 ppm and d at 7.89 ppm), 7.89 (d, 1 H, *J* = 1.3 Hz, ArH), 7.96 (d, 1 H, *J* = 7.9 Hz, ArH); ¹³C-NMR (DMSO-*d₆*, 150.9 MHz): δ (ppm) = 127.1, 128.9, 129.0, 129.7, 130.8, 132.3, 133.3 (tert. C); 90.7, 99.3, 112.0, 114.6, 120.8, 131.4, 133.5, 133.7, 140.9, 151.2, 152.3, 159.2, 166.3, 184.5 (quart. C); C₂₁H₁₁BrCl₂N₄OS (518.21); calcd C 48.67, H 2.14, N 10.81, S 6.19; found C 48.56, H 2.33, N 10.91, S 6.17; HPLC: 99.0% at 254 nm and 99.2% at 280 nm, t_{N} = 4.55 min, t_{M} = 1.04 min (ACN/H₂O; 60:40), λₘₐₓ: 329 nm.

### Example 3: 3,6-diamino-2-(3-chlorobenzoyl)-4-(2-chlorophenyl)thieno[2,3-b]pyridine-5-carbonitrile (compound No. 22 of the Table)

According to general procedure 2 from 6-amino-4-(2-chlorophenyl)-2-thioxo-1,2-dihydropyridine-3,5-dicarbonitrile (compound of formula (III)) (74.6 mg, 0.260 mmol), 10% aqueous solution of potassium hydroxide (146 µl twice, 0.520 mmol) and 2-bromo-1-(3-chlorophenyl)ethanone (60.6 mg, 0.260 mmol). Reaction period: 3.5 h. Crystallization from ethanol afforded 42.3 mg (37%) of an orange powder.

mp.: 289-290°C; IR (KBr): 3477 cm⁻¹ and 3362 cm⁻¹ (NH), 2216 cm⁻¹ (C≡N); ¹H-NMR (DMSO-*d₆*, 600 MHz): δ (ppm) = 6.69 (br s, 2 H, NH₂), 7.54-7.56 (m, 1 H, ArH), 7.62-7.64 (m, 1 H, ArH), 7.65-7.67 (m, 4 H, ArH), 7.69-7.72 (m, 1 H, ArH), 7.76-7.91 (m, 3 H, NH₂ and ArH overlapping); ¹³C-NMR (DMSO-*d₆*, 150.9 MHz): δ (ppm) = 125.7, 126.9, 128.6, 130.0, 130.4, 130.6, 130.8, 132.5 (tert. C); 90.9, 99.6, 112.4, 114.8, 131.1, 131.8, 133.4, 142.7, 150.8, 151.2, 159.3, 166.4, 185.9 (quart. C); C₂₁H₁₂Cl₂N₄OS (439.32); calcd C 57.41, H 2.75, N 12.75, S 7.30; found C 57.23, H 2.93, N 12.67, S 7.34; HPLC: 98.0% at 254 nm and 98.0% at 280 nm, t_{N} = 5.39 min, t_{M} = 1.03 min (ACN/H₂O; 55:45), λₘₐₓ: 328 nm.

### Example 4: 3,6-diamino-4-(2-chlorophenyl)-2-(3-cyanobenzoyl)thieno[2,3-blpyridine-5-carbonitrile (compound No. 26 of the Table))

According to general procedure 2 from 6-amino-4-(2-chlorophenyl)-2-thioxo-1,2-dihydropyridine-3,5-dicarbonitrile (compound of formula (III)) (86.3 mg, 0.301 mmol), 10% aqueous solution of potassium hydroxide (168 µl twice, 0.600 mmol) and 3-(bromoacetyl)benzonitrile (67.4 mg, 0.301 mmol). Reaction period: 1 h. Crystallization from ethanol afforded 44.2 mg (34%) of a yellow powder.

mp.: 303-305°C; IR (KBr): 3478 cm⁻¹ and 3390 cm⁻¹ (NH), 2217 cm⁻¹ (C=N); ¹H-NMR (DMSO-*d*₆, 600 MHz): δ (ppm) = 6.71 (br s, 2 H, NH₂), 7.64-7.67 (m, 2 H, ArH), 7.70-7.75 (m, 2 H, ArH), 7.82 (d, 1 H, *J* = 7.9 Hz, ArH, overlapped by br s at 7.87 ppm), 7.87 (br s, 2 H, NH₂), 7.99-8.04 (m, 2 H, ArH), 8.10-8.11 (m, 1 H, ArH); ¹³C-NMR (DMSO-*d*₆, 150.9 MHz): δ (ppm) = 128.4, 129.8, 129.8, 130.3, 130.6, 131.5, 132.3, 134.3 (tert. C); 90.8, 99.4, 111.6, 112.1, 114.7, 118.1, 130.9, 131.6, 141.6, 150.8, 151.3, 159.2, 166.3, 185.2 (quart. C); C₂₂H₁₂ClN₅OS (429.88); calcd C 61.47, H 2.81, N 16.29, S 7.46; found C 61.63, H 2.89, N 16.28, S 7.33; HPLC: 97.5% at 254 nm and 97.6% at 280 nm, t_{N} = 2.30 min, t_{M} = 1.03 min (ACN/H₂O; 50:50), λₘₐₓ: 328 nm.

### Example 5: 3,6-diamino-4-(2-bromophenyl)-N-(4-chlorophenyl)-5-cyanothieno[2,3-b]pyridine-2-carboxamide (Compound No.33 of the Table)

According to general procedure 2 from 6-amino-4-(2-bromophenyl)-2-thioxo-1,2-dihydropyridine-3,5-dicarbonitrile (compound of formula (III)) (132.5 mg, 0.400 mmol), 10% aqueous solution of potassium hydroxide (224 µl twice, 0.800 mmol) and 2-chloro-*N*-(4-chlorophenyl)acetamide (81.6 mg, 0.400 mmol). Reaction period: 1 h. Crystallization from ethanol afforded 91.7 mg (46%) of a yellow powder.

mp.: 297-298°C; IR (KBr): 3482 cm⁻¹, 3459 cm⁻¹, 3400 cm⁻¹ and 3322 cm⁻¹ (NH), 3171 cm⁻¹ (CH aromat), 2212 cm⁻¹ (C≡N), 1631 cm⁻¹ (C=O); ¹H-NMR (DMSO-*d*₆, 600 MHz): δ (ppm) = 5.69 (br s, 2 H, NH₂), 7.33-7.35 (m, 2 H, part of a AA'XX' system, ArH), 7.52-7.60 (br s, 2 H, NH₂ overlapped by m, 1 H, ArH), 7.62 (dd, 1 H, *J* = 7.5/1.9 Hz, ArH), 7.64-7.67 (m, 3 H, ArH), 7.92 (dd, 1 H, *J* = 8.1/1.1 Hz, ArH), 9.46 (s, 1 H, NH); ¹³C-NMR (DMSO-*d*₆, 150.9 MHz): δ (ppm) = 121.2 (2 C), 126.9 (2 C), 127.4, 128.7, 130.8, 131.8 (tert. C); 88.9, 91.3, 112.3, 113.8, 119.9, 125.6, 133.0, 136.6, 146.0, 149.6, 157.3, 162.3, 162.5 (quart. C); C₂₁H₁₃BrCN₅OS (498.78); calcd C 50.57, H 2.63, N 14.04, S 6.43; found C 50.24, H 2.72, N 14.35, S 6.11; HPLC: 99.4% at 254 nm and 99.8% at 280 nm, t_{N} = 3.79 min, t_{M} = 1.02 min (ACN/H₂O; 60:40), λₘₐₓ: 318 nm, 282 nm, 385 nm.

### Example 6: 3,6-diamino-4-(2-methylphenyl)thieno[2,3-b]pyridine-2,5-dicarbonitrile (Compound No. 40 of the Table)

According to general procedure 2 from 6-amino-4-(2-methylphenyl)-2-thioxo-1,2-dihydropyridine-3,5-dicarbonitrile (compound of formula (III)) (134 mg, 0.502 mmol), 10% aqueous solution of potassium hydroxide (281 µl twice, 1.00 mmol) and chloroacetonitrile (31.6 µl, 0.502 mmol). Reaction period: 40 min. Yield: 119 mg (78%) of a yellow powder.

mp.: 253-255°C; IR (KBr): 3463 cm-1, 3329 cm-1 and 3225 cm-1 (NH), 2217 cm-1 and 2194 cm-1 (C≡N); 1H-NMR (DMSO-*d₆*, 400 MHz): δ (ppm) = 2.08 (s, 3 H, CH₃), 5.21 (s, 2 H, NH₂), 7.37 (dd, 1 H, J = 7.6/1.5 Hz, ArH), 7.43-7.47 (m, 1 H, ArH), 7.48-7.56 (m, 2 H, ArH), 7.60 (br s, 2 H, NH₂); 13C-NMR (DMSO-*d₆*, 100.6 MHz): δ (ppm) = 18.8 (CH₃); 126.7, 128.0, 130.3, 130.6 (tert. C); 68.3, 90.5, 111.6, 114.9, 115.4, 132.3, 135.0, 150.1, 151.9, 158.8, 164.6 (quart. C); C₁₆H₁₁N₅S (305.36); calcd C 62.93, H 3.63, N 22.93, S 10.50; found C 62.74, H 3.64, N 22.76, S 10.35; HPLC: 97.5% at 254 nm and 98.7% at 280 nm, t_{N} = 4.61 min, t_{M} = 1.02 min (ACN/H₂O; 40:60), λₘₐₓ: 302 nm, 369 nm.

### Example 7: 3,6-diamino-4-(2-chlorophenyl)thieno[2,3-b]pyridine-2,5-dicarbonitrile (Compound No 38 of the Table)

According to general procedure 2 from 6-amino-4-(2-chlorophenyl)-2-thioxo-1,2-dihydropyridine-3,5-dicarbonitrile (compound of formula (III)) (165 mg, 0.574 mmol), 10% aqueous solution of potassium hydroxide (321 µl twice, 1.15 mmol) and chloroacetonitrile (36.1 µl, 0.574 mmol). Reaction period: 1.5 h. Crystallization from ethanol afforded 35.2 mg (19%) of a yellow powder.

mp.: 259-262°C; IR (KBr): 3468 cm⁻¹, 3324 cm⁻¹ and 3221 cm⁻¹ (NH), 2218 cm⁻¹ and 2196 cm⁻¹ (C≡N); ¹H-NMR (DMSO-*d*₆, 400 MHz): δ (ppm) = 5.23 (s, 2 H, NH₂), 7.60-7.71 (m, 5 H, ArH and NH₂ overlapping), 7.78 (d, 1 H, *J* = 8.1 Hz, ArH); ¹³C-NMR (DMSO-*d*₆, 150.9 MHz): δ (ppm) = 128.2, 130.1, 130.2, 132.4 (tert. C); 69.1, 90.8, 111.7, 114.7, 115.3, 131.4, 131.6, 149.3, 149.9, 158.7, 164.7 (quart. C); C₁₅H₈ClN₅S (325.78); calcd C 55.30, H 2.48, N 21.50, S 9.84; found C 55.47, H 2.77, N 21.31, S 9.54; HPLC: 96.1% at 254 nm and 97.4% at 280 nm, t_{N} = 4.63 min, t_{M} = 1.02 min (ACN/H₂O; 40:60), λₘₐₓ: 303 nm, 280 nm, 375 nm.

### Example 8: [3-amino-4-(2-iodophenyl)-5,6,7,8-tetrahydrothieno[2,3-b]quinoline-2-yl](4-chlorophenyl)methanone (Compound No. 44 of the Table)

According to general procedure 2 from 4-(2-iodophenyl)-2-thioxo-1,2,5,6,7,8-hexahydroquinoline-3-carbonitrile (compound of formula (III)) (51.2 mg, 0.131 mmol), 10% aqueous solution of potassium hydroxide (73.4 µl twice, 0.262 mmol) and 2-bromo-l-(4-chlorphenyl)ethanone (30.9 mg, 0.132 mmol). Reaction period: 40 min. Yield: 35.1 mg (49%) of a yellow powder.

mp.: 211-212°C; IR (KBr): 3469 cm⁻¹ (NH), 2928 cm⁻¹ (CH aliph); ¹H-NMR (DMSO-*d*₆, 600 MHz): δ (ppm) = 1.66-1.77 (m, 2 H, CH₂), 1.79-1.88 (m, 2 H, CH₂), 2.20-2.32 (m, 2 H, CH₂), 2.97-3.06 (m, 2 H, CH₂), 6.56 (br s, 2 H, NH₂), 7.36 ("dt", 1 H, *J* = 7.9/1.5 Hz, ArH), 7.45 (dd, 1 H, *J* = 7.5/1.1 Hz, ArH), 7.60-7.62 (m, 2 H, part of a AA'XX' system, ArH), 7.67 ("dt", 1 H, *J* = 7.5/0.6 Hz, ArH); 7.77-7.79 (m, 2 H, part of a AA'XX' system, ArH), 8.15 (d, 1 H, *J* = 7.9 Hz, ArH); ¹³C-NMR (DMSO-*d*₆, 150.9 MHz): δ (ppm) = 21.9 (2 C), 25.8, 33.1 (sek. C); 128.5 (2 C), 128.7, 129.1 (2 C), 129.4, 131.0, 139.3 (tert. C); 98.4, 102.3, 119.1, 126.6, 135.7, 139.2, 139.3, 148.2, 150.3, 158.7, 161.2, 187.4 (quart. C); C₂₄H₁₈ClN₂OS (544.84); calcd C 52.91, H 3.33, N 5.14, S 5.89; found C 52.84, H 3.41, N 5.33, S 5.82; HPLC: 97.1% at 254 nm and 97.3% at 280 nm, t_{N} = 5.84 min, t_{M} = 1.08 min (ACN/H₂O; 80:20), λₘₐₓ: 283 nm, 321 nm.

### Example 9: 7-amino-9-(2-chlorophenyl)-4-oxo-3,4-dihydropyrido[3',2':4,5]thieno[3,2-d]pyrimidine-8-carbonitrile (Compound 46 of the table)

A suspension of 3,6-diamino-4-(2-chlorophenyl)thieno[2,3-b]pyridine-2,5-dicarbonitrile (compound 38 of the table) (97.7 mg, 0.300 mmol) in formic acid was refluxed for 5 h. After cooling to room temperature a precipitate was collected by suction. Crystallization from ethanol afforded 34.8 mg (33%) of a yellow powder.

mp.: 377-378°C; IR (KBr): 3464 cm⁻¹, 3332 cm⁻¹ and 3209 cm⁻¹ (NH), 2217 cm-1 (C≡N), 1657 cm-1 (C=O); 1H-NMR (DMSO-*d₆*, 600 MHz): δ (ppm) = 7.47-7.50 (m, 2 H, ArH), 7.54-7.57 (m, 1 H, ArH), 7.62-7.64 (m, 1 H, ArH), 7.75 (br s, 2 H, NH2), 7.96 (s, 1 H, pyrimidine-CH), 12.75 (s, 1 H, NH); 13C-NMR (DMSO-*d₆*, 150.9 MHz): δ (ppm) = 125.9, 127.9, 129.1, 129.7, 146.3 (tert. C); 90.4, 113.9, 115.0, 116.8, 130.4, 132.4, 149.6, 150.6, 155.6, 157.9, 164.9 (quart. C); Cₗ₆HgC1N₅0S (353.79); calcd C 54.32, H 2.28, N 19.80, S 9.06; found C 54.12, H 2.41, N 19.52, S 8.82; HPLC: 98.1% at 254 nm and 98.7% at 280 nm, t_{N} = 5.06 min, t_{M} = 1.03 min (ACN/H2O; 30:70), λₘₐₓ: 236 nm, 281 nm, 356 nm.

### Example 10: 7 -amino-9-(2-chlorophenyl)-4-thioxo-3,4-dihydropyrido[3',2':4,5]thieno[3,2-d]pyrimidine-8-carbonitrile (compound 47 of the table)

7-amino-9-(2-chlorophenyl)-4-oxo-3,4-dihydropyrido[3',2' :4,5]-thieno[3,2-d]pyrimidine-8-carbonitrile (compound 46) (24.0 mg, 0.068 mmol) and Lawessons reagent (27.5 mg, 0.068 mmol) were suspended in 1,4-dioxane (0.23 ml) and refluxed for 30 min. After cooling to room temperature the solvent was evaporated in vacuo and the residue was suspended in ethylacetate. The solid was collected by suction. Crystallization from ethanol afforded 11.5 mg (46%) of a yellow powder.

mp.: 305°C beginning decomposition, 319°C black melting; IR (KBr): 3421 cm⁻¹, 3315 cm⁻¹ and 3211 cm⁻¹ (NH), 2223 cm⁻¹ (C=N); 1H-NMR (DMSO-*d₆*, 600 MHz): δ (ppm) = 7.49-7.53 (m, 2 H, ArH), 7.55-7.58 (m, 1 H, ArH), 7.64-7.65 (m, 1 H, ArH), 7.97 (br s, 2 H, NH2, deleted with D2O), 8.16 (d, 1 H, J = 3.6 Hz, pyrimidine-CH, s with D₂O (coupling with br s at 14.22 ppm)), 14.22 (s, 1 H, NH, deleted with D₂O); 13C-NMR (DMSO-*d₆*, 150.9 MHz): δ (ppm) = 127.2, 129.1, 130.2, 131.0, 146.9 (tert. C); 91.8, 114.8, 115.8, 131.4, 132.3, 133.2, 147.2, 151.7, 159.3, 167.4, 174.5 (quart. C); C1₆HgClN₅S₂ (369.85); calcd C 51.96, H 2.18, N 18.94, S 17.34; found C 52.29, H 2.26, N 19.19, S 16.91; HPLC: 98.5% at 254 nm and 98.3% at 280 nm, t_{N} = 2.25 min, t_{M} = 1.03 min (ACN/H₂O; 40:60), λₘₐₓ: 244 nm, 313 nm, 382 nm.

The following table illustrates the chemical structures and the physical properties of some compounds according to the invention.

**TABLE**

| | | | | | | |
|---|---|---|---|---|---|---|
| 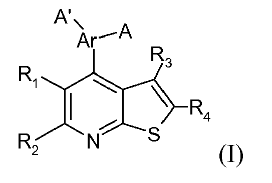 | | | | | | |

| **No (compound number)** | **R₁** | **R₂** | **R₃** | **R₄** | 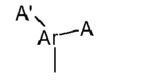 | **Melting point (°C)** |
|---|---|---|---|---|---|---|
| 1 | -CN | -NH₂ | -NH₂ | 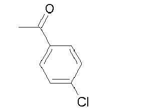 | 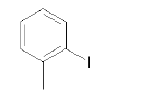 | 358-361 |
| 2 | -CN | -NH₂ | -NH₂ | 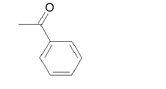 | 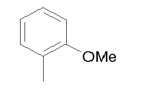 | 263-264 |
| 3 | -CN | -NH₂ | -NH₂ | 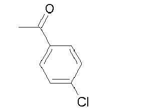 | 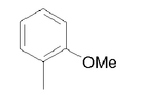 | 317-319 |
| 4 | -CN | -NH₂ | -NH₂ | 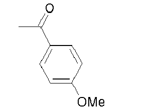 | 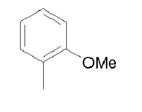 | 245-247 |
| 5 | -CN | -NH₂ | -NH₂ | 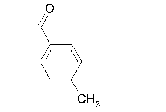 | 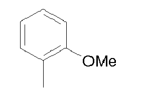 | 245-246 |
| 6 | -CN | -NH₂ | -NH₂ | 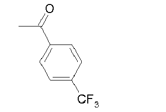 | 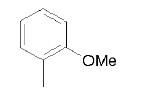 | 253-254 |
| 7 | -CN | -NH₂ | -NH₂ | 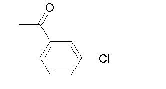 | 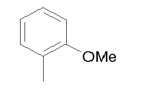 | 253-256 |
| 8 | -CN | -NH₂ | -NH₂ | 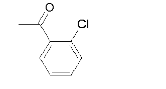 | 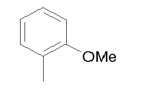 | 260-262 |
| 9 | -CN | -NH₂ | -NH₂ | 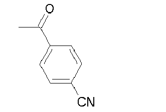 | 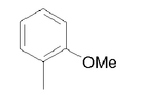 | 338-339 |
| 10 | -CN | -NH₂ | -NH₂ | 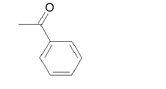 | 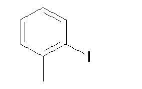 | 325-326 |
| 11 | -CN | -NH₂ | -NH₂ | 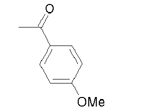 | 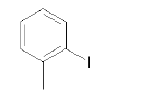 | 316-317 |
| 12 | -CN | -NH₂ | -NH₂ | 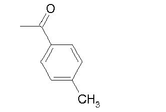 | 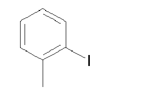 | 328-330 |
| 13 | -CN | -NH₂ | -NH₂ | 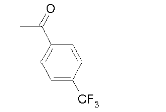 | 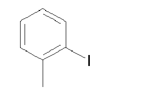 | 306-307 |
| 14 | -CN | -NH₂ | -NH₂ | 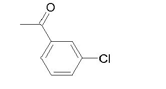 | 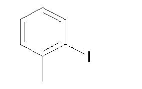 | 275-277 |
| 15 | -CN | -NH₂ | -NH₂ | 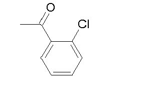 | 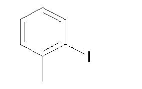 | 288-289 |
| 16 | -CN | -NH₂ | -NH₂ | 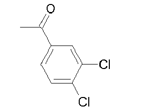 | 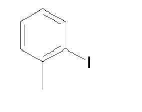 | 286-289 |
| 17 | -CN | -NH₂ | -NH₂ | 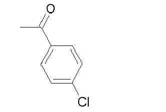 | 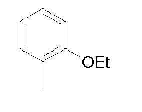 | 256-257 |
| 18 | -CN | -NH₂ | -NH₂ | 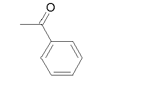 | 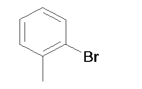 | 350-351 |
| 19 | -CN | -NH₂ | -NH₂ | 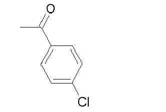 | 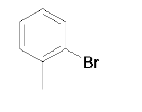 | 355-358 |
| 20 | -CN | -NH₂ | -NH₂ | 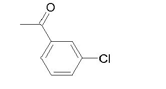 | 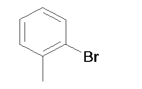 | 280-283 |
| 21 | -CN | -NH₂ | -NH₂ | 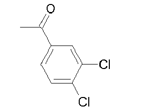 | 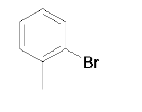 | 289-290 |
| 22 | -CN | -NH₂ | -NH₂ | 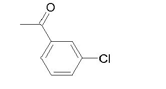 | 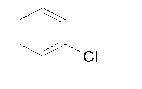 | 289-290 |
| 23 | -CN | -NH₂ | -NH₂ | 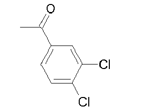 | 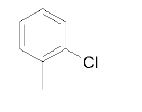 | 273-275 |
| 24 | -CN | -NH₂ | -NH₂ | 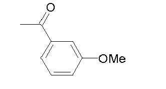 | 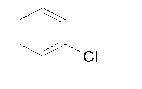 | 224-225 |
| 25 | -CN | -NH₂ | -NH₂ | 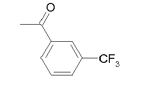 | 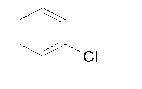 | 255-257 |
| 26 | -CN | -NH₂ | -NH₂ | 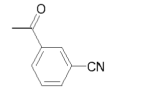 | 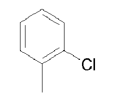 | 303-305 |
| 27 | -CN | -NH₂ | -NH₂ | 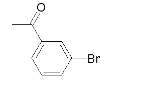 | 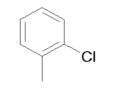 | 267-268 |
| 28 | -CN | -NH₂ | -NH₂ | 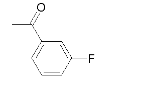 | 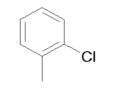 | 352-353 |
| 29 | -CN | -NH₂ | -NH₂ | 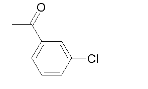 | 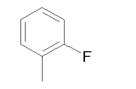 | 284-285 |
| 30 | -CN | -NH₂ | -NH₂ | 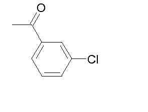 | 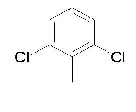 | 278-279 |
| 31 | -CN | -NH₂ | -NH₂ | 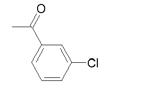 | 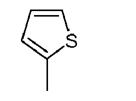 | 277-279 |
| 32 | -CN | -NH₂ | -NH₂ | 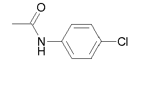 | 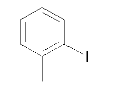 | 270-271 |
| 33 | -CN | -NH₂ | -NH₂ | 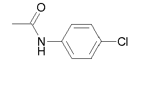 | 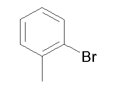 | 297-298 |
| 34 | -CN | -NH₂ | -NH₂ | 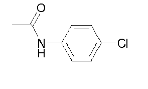 | 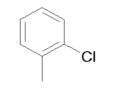 | 306-307 |
| 35 | -CN | -NH₂ | -NH₂ | -CN | 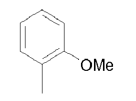 | 303-304 |
| 36 | -CN | -NH₂ | -NH₂ | -CN | 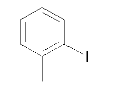 | 272-273 |
| 37 | -CN | -NH₂ | -NH₂ | -CN | 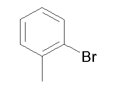 | 262-263 |
| 38 | -CN | -NH₂ | -NH₂ | -CN | 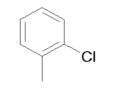 | 259-262 |
| 39 | -CN | -NH₂ | -NH₂ | -CN | 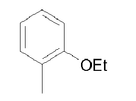 | 270-271 |
| 40 | -CN | -NH₂ | -NH₂ | -CN | 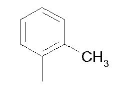 | 253-255 |
| 41 | -CN | -NH₂ | -NH₂ | -CN | 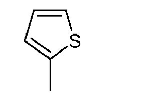 | 309-310 |
| 42 | -CN | -NH₂ | -NH₂ | -CN | 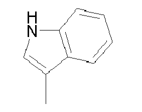 | 293-316 |
| 43 | 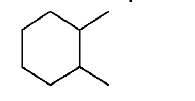 | | -NH₂ | 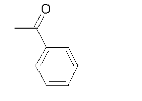 | 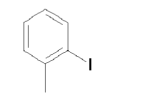 | 216-218 |
| 44 | 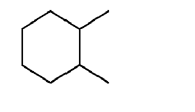 | | -NH₂ | 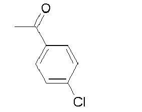 | 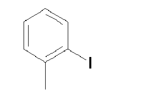 | 211-212 |
| 45 | 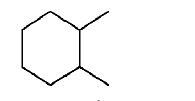 | | -NH₂ | 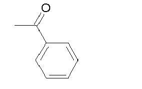 | 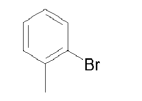 | 219-221 |
| 46 | -CN | -N | 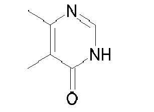 | | 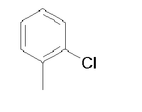 | 377-378 |
| 47 | -CN | -N | 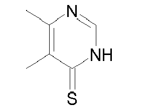 | | 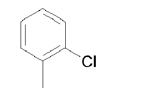 | 305 |

In this table:
- Me represents a methyl group,
- Et represents an ethyl group,
- OMe represents a methoxy group, and
- OEt represents an ethoxy group.

### Example 11: Pharmacological test

The compounds of the invention have been the subject of pharmacological tests which have demonstrated their relevance as active substances against *Pf*GSK-3.

Malaria is a generic term covering infection by several Plasmodium species, namely *P. falciparum, P. vivax, P. ovale, P. malariae* and *P. knowlesi.*

The inhibitory activity of the compounds of formula (I) according to the present invention was restricted to GSK-3 from *Plasmodium* species.

### Example 11.1: An inhibitory scaffold specific for PfGSK-3 versus mammalian GSK-3

Some of the compounds of formula (I) according to the present invention display both sub-micromolar potency on *Pf*GSK-3 and exquisite selectivity towards *Pf*GSK-3 versus mammalian GSK-3(α/β. The compounds of formula (I) according to the present invention appear as unique *Pf*GSK-3 selective inhibitors which are essentially devoid of activity towards the mammalian GSK-3 isoforms.

### MATERIAL AND METHODS

### Expression and purification of recombinant (His)₆-tagged PfGSK3

*Escherichia coli* strain (TOP 10) containing a plasmid encoding PfGSK3 with six histidines (pBAD-Thio-TOPO, Invitrogen) were cultured into 500 ml of LB medium containing 100 µg/ml ampicillin, at 37°C, under shaking (250 rpm) until OD₆₀₀ of 0.5 was reached. Expression was induced with 0.5 ml of 20% arabinose (0.02% final concentration). The culture was grown at 37°C with shaking for 4 hours. Cells were harvested by centrifugation at 9000 rpm for 10 min. The bacterial pellet was stored frozen at -80°C until further processing. For proteins extraction, pellet was resuspended in 10 ml of lysis buffer (50 mM NaH₂PO₄ pH 8.0, 300 mM NaCl, 10 mM imidazole) with the addition of protease inhibitors (complete EDTA-free, Roche) and lysozyme (300 mg/ml final). Cells were disrupted by sonication and the cell lysate was centrifuged at 24000 rpm for 30 min at 4°C. The recombinant *Pf*GSK3 protein was purified by Immobilized Metal Affinity Chromatography according to a batch procedure on 1 ml of Nickel affinity resin (Ni-NTA agarose beads, Qiagen). Beads were washed four times with 10 ml of wash buffer (50 mM NaH₂PO₄ pH 8.0, 300 mM NaCl, 30 mM imidazole). Protein was eluted with one volume of elution buffer (50 mM NaH₂PO₄ pH 8.0, 300 mM NaCl, 250 mM imidazole, 10% glycerol).

### Purification of native porcine GSK3

Porcine GSK3 (GSK-3α/β) was purified from porcine brain by affinity chromatography on immobilized axin according to the protocol described in Primot et al (Protein Expression and Purification 20, 394-404, 2000).

### Kinase assay

Both porcine GSK3 and *Pf*GSK3 were assayed with 40 µM GS-1 peptide, a specific GSK-3 substrate, (YRRAAVPPSPSLSRHSSPHQSpEDEEE, synthesized by the Peptide Synthesis Unit, Institute of Biomolecular Sciences, University of Southampton, U.K.), in buffer A (25 mM Tris-HCl pH 7.5, 10 mM MgC12, 1 mM EGTA, 1 mM DTT, 50 µg heparin/ml, 0.5 mg BSA/ml) in the presence of 15 µM [γ-³³P] ATP (3000 Ci/mmol; 1 mCi/ml) in a final volume of 30 µl. After 30 min. incubation at 30°C, 25 µl aliquots of supernatant were spotted onto 2.5 x 3 cm pieces of Whatman P81 phosphocellulose paper, and, 20 sec. later, the filters were washed five times (for at least 5 min. each time) in 1 % phosphoric acid. The wet filters were counted in the presence of 1 ml ACS (Amersham) scintillation fluid. The kinase activity was expressed in pmoles phosphate incorporated in GS-1 or in percentage of the maximal activity.

### RESULTS

Each of the tested compounds was effective at inhibiting *Pf*GSK-3 and not mammalianGSK-3. In particular a large amount of compounds show a *Pf*GSK-3 IC₅₀ values ranging from 0.3 µM to 4 µM, and more particularly compounds 11, 21, 22, 26, 40, 41 and 47 of the table exhibit IC₅₀ values as expressed in the following table:

| Compound No | 11 | 21 | 22 | 26 | 40 | 41 | 47 |
|---|---|---|---|---|---|---|---|
| IC₅₀ *Pf*GSK-3 | 1.7 | 3.8 | 0.48/0.060 | 0.5 | 0.51 | 0.18 | 0.13 |
| IC₅₀ Mammalian GSK-3 | -(10) | -(10) | >10 | >10 | 2 | 2.3 | 0.06 |

### Example 11.2: Kinase Selectivity of compounds of formula (I) according to the present invention

Compound No. 22 of the Table was selected as a representative inhibitor of compounds of formula (I) according to the present inventionfor further investigations. Compound No. 22 shows major selectivity towards *Pf*GSK-3 (IC50: 0.47 µM) compared to mammalian GSK-3α/β (IC50: > 10 µM).

Compound No. 22 was also tested on a small panel of 22 purified kinases, including 4 *P. falciparum* kinases. Besides *Pf*GSK-3 no kinase was found to be inhibited by the compound at concentrations up to 10 µM. This lack of effect on all tested kinases was also shown for other PfGSK-3 inhibitors according to the present invention. To complement this selectivity study, compound No 22 was run on two large scale selectivity panels: Prof. P. Cohen's kinase selectivity panel (76 kinases) (Bain J, Plater L, Elliott M, Shapiro N, Hastie CJ, McLauchlan H et al. (2007). The selectivity of protein kinase inhibitors: a further update. Biochem J. 408: 297-315.) and the Ambit Biosciences interaction assay (402 kinases) (Karaman MW, Herrgard S, Treiber DK, Gallant P, Atteridge CE, Campbell BT, Chan KW, Ciceri P, Davis MI, Edeen PT, Faraoni R, Floyd M, Hunt JP, Lockhart DJ, Milanov ZV, Morrison MJ, Pallares G, Patel HK, Pritchard S, Wodicka LM, Zarrinkar PP. A quantitative analysis of kinase inhibitor selectivity. Nat Biotechnol. 2008 Jan;26(1):127-32.).

### RESULTS

All these selectivity panels and comparison with rather selective (roscovitine) and unselective (staurosporine) kinase inhibitors showed that Compound No 22 is globally a very poor, if at all, inhibitor of mammalian kinases.

### Example 11.3: Species specificity of the compounds of formula (I) according to the present invention towards GSK-3 orthologues of Plasmodium

Given the strong conservation of GSK-3 across evolution and the surprising selectivity of the compounds according to the present invention towards PfGSK-3 versus mammalian GSK-3, a selection of 12 GSK-3 orthologues of various species chosen on the "tree of life" analyzed. The species belong to the Apicomplexa (*P. falciparum, P. vivax, P. knowlesi* and *P. berghei* (Gardner, M.J. et al. Genome sequence of the human malaria parasite Plasmodium falciparum. Nature 419, 498-511 (2002); Carlton, J.M. et al. Comparative genomics of the neglected human malaria parasite Plasmodium vivax. Nature 455, 799-803 (2008); Pain, A. et al. The genome of the simian and human malaria parasite Plasmodium knowlesi. Nature 455, 799-803 (2008).). *Toxoplasma gondii* (Qin, C.L., Tang, J. and Kim, K. Cloning and in vitro expression of TPK3, a Toxoplasma gondii homologue of shaggy/glycogen synthase kinase-3 kinases. Mol. Biochem. Parasitol. 93, 273-283 (1998)), *Cryptosporidium parvum. Euglenozoa* (*Leishmania donovani, L. major, Trypanosoma brucei* (Ojo, K.K. et al. Glycogen synthse kinase 3 is a potential drug target for African trypanosomiasis. Antimicrobiol. Agents Chemother. 52, 3710-3717 (2008).), *T. cruzi, Amoebozoa* (*Dictyostelium discoideum*) and *metazoa* (*Anopheles gambiae*, *Sus crofa*, *Homo sapiens*). Comparison of the primary sequences of these GSK-3 orthologues showed that their divergence coincided with species divergence in evolution.

### MATERIAL AND METHODS

Following PCR amplification from cDNA libraries or direct synthesis based on databases sequences, the GSK-3 genes were inserted in appropriate expression vectors and recombinant, tagged kinases were expressed, affinity purified and assayed in the presence of up to 10-100µM of each compound of formula (I) according to the present invention and a selection of the *Pf*GSK-3 inhibitors.

### RESULTS

The inhibitory activity of the compounds of formula (I) according to the present invention was found only in *Plasmodium* GSK-3.

More particularly, six compounds have given the following results gathered in a table. IC5o values, calculated from dose-response curves, are presented in µM.

| Species/compounds No. | 20 | 22 | 26 | 2 | 1 | 14 |
|---|---|---|---|---|---|---|
| *Plasmodium falciparum* | 0.47 | 0.41 | 0.21 | 7.8 | 1.9 | 101 |
| *Plasmodium knowlesi* | 1.45 | 2.45 | 6.5 | 95.0 | 15.5 | 2.75 |
| *Toxaplasma grondii* | -(100) | -(100) | -(100) | -(100) | -(100) | -(100) |
| *Cryptosporidium parvum* | -(100) | -(100) | -(100) | -(100) | -(100) | -(100) |
| *Trypanopsoma cruzi* | -(100) | -(100) | -(100) | -(100) | -(100) | -(100) |
| *Leishmania brucei* | -(100) | -(100) | -(100) | -(100) | -(100) | -(100) |
| *Leishmania majo* | -(100) | -(100) | -(100) | -(100) | -(100) | -(100) |
| *Leishmania donovani* | 44 | 72 | 100 | 80 | 80 | 70 |
| *Dictyostelium discoideum* | 80 | > 10 | > 100 | -(100) | -(100) | > 100 |
| *Sus scrofa (GSK-3* α/β*)* | 5.6 | > 10 | 22 | -(100) | -(100) | -(100) |
| *Homo sapiens (GSK-3* α*)* | > 100 | > 100 | > 25 | -(100) | -(100) | -(100) |
| *Homo sapiens (GSK- 3* β*)* | 4.0 | 3.3 | 8.1 | >1400 | -(100) | 50 |

Therefore, the results of the tests carried out on the compounds disclosed in the present invention show that, they exhibit an antimalarial activity.

For this purpose an effective amount of a said compound may be administered to a subj ect suffering from malaria.

According to another of its aspects, the present invention relates to pharmaceutical compositions containing an active ingredient, chosen among a compound of formula (I), (Ia), (I'a), (Ib), (I'b), (Ic), (Id) or anyone of the compounds (1) to (47) their salts with pharmaceutically acceptable acids and mixture thereof.

Thus, these pharmaceutical compositions contain an effective amount of said compound, and one or more pharmaceutical excipients.

The aforementioned excipients are selected according to the dosage form and the desired mode of administration.

In this context the compounds according to the present invention can be present in any pharmaceutical form which is suitable for enteral or parenteral administration, in association with appropriate excipients, for example in the form of plain or coated tablets, hard gelatine and other capsules, suppositories, or drinkable or injectable solutions or suspensions, in doses which enable the daily administration of from 0.1 to 1000 mg of active substance.

The present invention according to another of its aspects, also relates to a method of preventing, inhibiting or treating pathological or nonpathological conditions linked with malaria which comprises administering to a subject suffering there from an effective amount of a compound according to formula (I), anyone of compounds of formula (Ia), (I'a), (Ib), (I'b), (Ic), (Id) according to the invention or anyone of the individual compounds (1) to (47) as listed above in the table or one of its pharmaceutically acceptable salts.

The present invention according to another of its aspects, also relates to a method of preventing, inhibiting or treating pathological or nonpathological conditions linked with malaria which comprises administering to a subject suffering there from an effective amount of a compound according to formula (I), anyone of compounds of formula (Ia), (I'a), (Ib), (I'b), (Ic) or (Id) according to the invention or anyone of the individual compounds (1) to (47) as listed above or one of its pharmaceutically acceptable salts in combination with at least one other anti-malaria agent for a therapeutic use.

## Claims

1. Compound of formula (I) 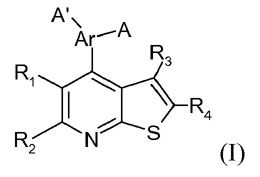 wherein
**R₁** represents a -CN group and **R₂** represents a -NH₂ group or alternatively **R₁** and **R₂** together form a cyclcohexyl ring fused with the thieno[2,3-*b*]pyridine group,
**R₃** represents a -NH₂ group, or a NHCOR₇ group wherein R₇ is a (C₁-C₄) alkyl group,
**R₄** represents a -CN group or a formula (A) 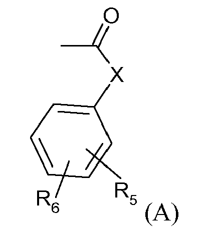 wherein
**R₅** and **R₆** independently represent a hydrogen atom, a halogen atom, a trifluoromethyl group, a -CN group, a (C₁-C₄)alkoxy or a (C₁-C₄)alkyl group, and
**X** represents a bond or a -NH- group,
wherein alternatively R₃ and R₄ together form a ring of formula (B), fused to the thieno[2,3-*b*]pyridine 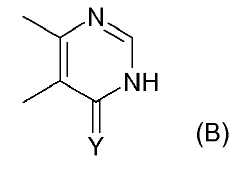 wherein
Y is an oxygen or a sulphur atom,
**Ar** represents a phenyl group, or a heteroaryl group chosen among thienyl, indolyl and furyl group,
**A** and **A'** independently represent a hydrogen atom, a (C₁-C₄)alkoxy group or a halogen atom, and
wherein when **Ar** is a phenyl group, at least one of A or A' is different form a hydrogen atom and is in the ortho position,
wherein (C₁-C₄)alkoxy group and (C₁-C₄)alkyl group may potentially independently be substituted by one group chosen among a -NH₂ group and a -COOH group,
with the exclusion of the following four compounds of formula (I) :
- (i) and (ii) wherein **Ar** is a phenyl group, **R₁** is a -CN group, **R₂** and **R₃** are each a -NH₂ group, **A** is a o-I or a o-Cl and **A'** is a hydrogen atom, **R₄** represents a formula (A) wherein **X** is a bond, **R₅** is a hydrogen atom and **R₆** is a p-Cl, and
- (iii) and (iv) wherein **Ar** is a furyl group, **R₁** is a -CN group, **R₂** and **R₃** are each a -NH₂ group, **A** and **A'** are hydrogen atom, **R₄** represents a formula (A) wherein **X** is a bond, **R₅** is a hydrogen atom and **R₆** is a p-Cl or a hydrogen atom,
or anyone of their salts with pharmaceutically acceptable acids and mixtures thereof.

2. Compound according to claim 1, wherein it has the following formula (Ia) 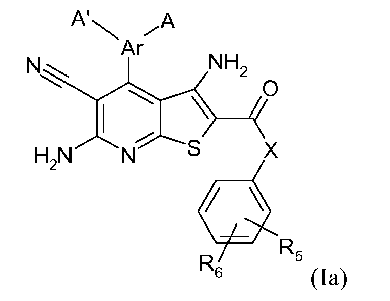 wherein
**Ar** represents a phenyl group or a 2-thienyl group,
**A** and **A'** independently represent a hydrogen atom, a (C₁-C₄)alkoxy group or a halogen atom,
**X** represents a bond or a -NH- group,
**R₅** and **R₆** independently represent a hydrogen atom, a halogen atom, a (C₁-C₄)alkoxy group, a (C₁-C₄)alkyl group, a trifluoromethyl group or a -CN group, and
wherein when **Ar** is a phenyl group, at least one of A or A' is different form a hydrogen atom and is in the ortho position, and
wherein (C₁-C₄)alkoxy group and (C₁-C₄)alkyl group may potentially independently be substituted by one group chosen among a -NH₂ group and a -COOH group,
with the exclusion of the following four compounds of formula (I) :
- (i) and (ii) wherein **Ar** is a phenyl group, **R₁** is a -CN group, **R₂** and **R₃** are each a -NH₂ group, **A** is a o-I or a o-Cl and **A'** is a hydrogen atom, **R₄** represents a formula (A) wherein **X** is a bond, **R₅** is a hydrogen atom and **R₆** is a p-Cl, and
- (iii) and (iv) wherein **Ar** is a furyl group, **R₁** is a -CN group, **R₂** and **R₃** are each a -NH₂ group, **A** and **A'** are hydrogen atom, **R₄** represents a formula (A) wherein **X** is a bond, **R₅** is a hydrogen atom and **R₆** is a p-Cl or a hydrogen atom.

3. Compound according to claim 1, wherein it has the following formula (I'a) 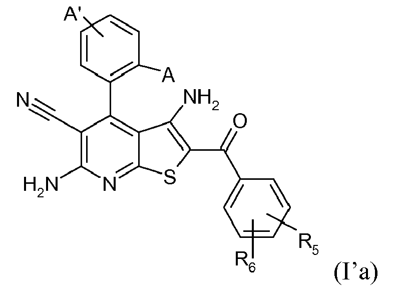 wherein
**A** represents a (C₁-C₄)alkoxy group or a halogen atom,
**A'** represents a hydrogen atom, a (C₁-C₄)alkoxy group or a halogen atom,
**R₅** and **R₆** independently represent a hydrogen atom, a halogen atom, a (C₁-C₄)alkoxy group, a(C₁-C₄)alkyl group, a trifluoromethyl group, a -CN group, and
wherein (C₁-C₄)alkoxy group and (C₁-C₄)alkyl group may potentially independently be substituted by one group chosen among a -NH₂ group and a -COOH group,
with the exclusion of the two compounds of formula (I) (i) and (ii) wherein **A** is a o-I or o-Cl and **A'** is a hydrogen atom, **R₅** is a hydrogen atom and **R₆** is a p-Cl.

4. Compound according to claim 1, wherein it has the following formula (Ib) 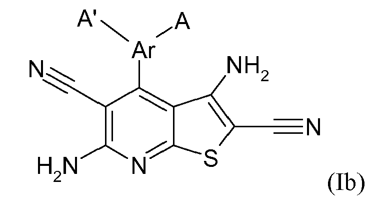 wherein
**Ar** represents a phenyl group, a 2-thienyl group or an indol-3-yl group,
**A** and **A'** independently represent a hydrogen atom, a (C₁-C₄)alkoxy group or a halogen atom, and
wherein (C₁-C₄)alkoxy group and (C₁-C₄)alkyl group may potentially independently be substituted by one group chosen among a -NH₂ group and a -COOH group, and
wherein when **Ar** is a phenyl group, at least one of A or A' is different from a hydrogen atom and is in the ortho position.

5. Compound according to claim 1, wherein it has the following formula (I'b) 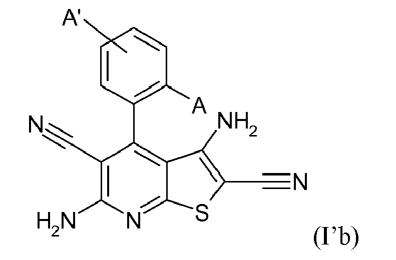 wherein
**A** represents a (C₁-C₄)alkoxy group or a halogen atom, and
**A'** represents a hydrogen atom, a (C₁-C₄)alkoxy group or a halogen atom, and
wherein (C₁-C₄)alkoxy group and (C₁-C₄)alkyl group may potentially independently be substituted by one group chosen among a -NH₂ group and a -COOH group.

6. Compound according to claim 1, wherein it has the following formula (Ic) 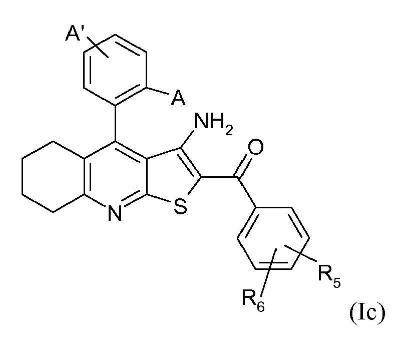 wherein
**A** represents a (C₁-C₄)alkoxy group or a halogen atom,
**A'** represents a hydrogen atom, a (C₁-C₄)alkoxy group or a halogen atom, and
**R₅** and **R₆** independently represent a hydrogen atom, a halogen atom, a (C₁-C₄)alkoxy group, a (C₁-C₄)alkyl group, a trifluoromethyl group, a -CN group, and
wherein (C₁-C₄)alkoxy group and (C₁-C₄)alkyl group may potentially independently be substituted by one group chosen among a -NH₂ group and a -COOH group.

7. Compound according to claim 1, wherein it has the following formula (Id) 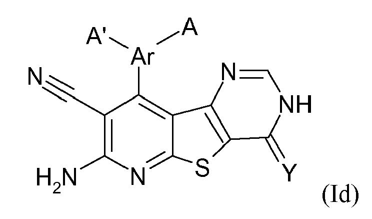 wherein
**Ar** represents a phenyl group or a 2-thienyl group,
**A** and **A'** independently represent a hydrogen atom, a (C₁-C₄)alkoxy group or a halogen atom, and
**Y** represents a sulphur or an oxygen atom,
wherein when **Ar** is a phenyl group, at least one of A or A' is different from a hydrogen atom and is in the orthoposition, and
wherein (C₁-C₄)alkoxy group and (C₁-C₄)alkyl group may potentially independently be substituted by one group chosen among a -NH₂ group and a -COOH group.

8. Compound according to claim 1, wherein it is chosen among:
- 3,6-diamino-2-benzoyl-4-(2-methoxyphenyl)thieno[2,3-*b*]pyridine-5-carbonitrile (2)
- 3,6-diamino-2-(4-chlorobenzoyl)-4-(2-methoxyphenyl)thieno[2,3-*b*]pyridine-5-carbonitrile (3)
- 3,6-diamino-2-(4-methoxybenzoyl)-4-(2-methoxyphenyl)thieno[2,3-b]pyridine-5-carbonitrile (4)
- 3,6-diamino-4-(2-methoxyphenyl)-2-(4-methylbenzoyl)thieno[2,3-*b*]pyridine-5-carbonitrile (5)
- 3,6-diamino-4-(2-methoxyphenyl)-2-[4-(trifluoromethyl)benzoyl]thieno[2,3-*b*]pyridine-5-carbonitrile (6)
- 3,6-diamino-2-(3-chlorobenzoyl)-4-(2-methoxyphenyl)thieno[2,3-*b*]pyridine-5-carbonitrile (7)
- 3,6-diamino-2-(2-chlorobenzoyl)-4-(2-methoxyphenyl)thieno[2,3-*b*]pyridine-5-carbonitrile (8)
- 3,6-diamino-2-(4-cyanobenzoyl)-4-(2-methoxyphenyl)thieno[2,3-*b*]pyridine-5-carbonitrile (9)
- 3,6-diamino-2-benzoyl-4-(2-iodophenyl)thieno[2,3-*b*]pyridine-5-carbonitrile (10)
- 3,6-diamino-4-(2-iodophenyl)-2-(4-methoxybenzoyl)thieno[2,3-*b*]pyridine-5-carbonitrile (11)
- 3,6-diamino-4-(2-iodophenyl)-2-(4-methylbenzoyl)thieno[2,3-*b*]pyridine-5-carbonitrile (12)
- 3,6-diamino-4-(2-iodophenyl)-2-[4-(trifluoromethyl)benzoyl]thieno[2,3-*b*]pyridine-5-carbonitrile (13)
- 3,6-diamino-2-(3-chlorobenzoyl)-4-(2-iodophenyl)thieno[2,3-*b*]pyridine-5-carbonitrile (14)
- 3,6-diamino-2-(2-chlorobenzoyl)-4-(2-iodophenyl)thieno[2,3-*b*]pyridine-5-carbonitrile (15)
- 3,6-diamino-2-(3,4-dichlorobenzoyl)-4-(2-iodophenyl)thieno[2,3-*b*]pyridine-5-carbonitrile (16)
- 3,6-diamino-2-(4-chlorobenzoyl)-4-(2-ethoxyphenyl)thieno[2,3-*b*]pyridine-5-carbonitrile (17)
- 3,6-diamino-2-benzoyl-4-(2-bromophenyl)thieno[2,3-*b*]pyridine-5-carbonitrile (18)
- 3,6-diamino-4-(2-bromophenyl)-2-(4-chlorobenzoyl)thieno[2,3-*b*]pyridine-5-carbonitrile (19)
- 3,6-diamino-4-(2-bromophenyl)-2-(3-chlorobenzoyl)thieno[2,3-*b*]pyridine-5-carbonitrile (20)
- 3,6-diamino-4-(2-bromophenyl)-2-(3,4-dichlorobenzoyl)thieno[2,3-*b*]pyridine-5-carbonitrile (21)
- 3,6-diamino-2-(3-chlorobenzoyl)-4-(2-chlorophenyl)thieno[2,3-*b*]pyridine-5-carbonitrile (22)
- 3,6-diamino-4-(2-chlorophenyl)-2-(3,4-dichlorobenzoyl)thieno[2,3-*b*]pyridine-5-carbonitrile (23)
- 3,6-diamino-4-(2-chlorophenyl)-2-(3-methoxybenzoyl)thieno[2,3-*b*]pyridine-5-carbonitrile (24)
- 3,6-diamino-4-(2-chlorophenyl)-2-[3-(trifluoromethyl)benzoyl]thieno[2,3-*b*]pyridine-5-carbonitrile (25)
- 3,6-diamino-4-(2-chlorOphenyl)-2-(3-cyanobenzoyl)thieno[2,3-*b*]pyridine-5-carbonitrile (26)
- 3,6-diamino-2-(3-bromobenzoyl)-4-(2-chlorophenyl)thieno[2,3-*b*]pyridine-5-carbonitrile (27)
- 3,6-diamino-4-(2-chlorophenyl)-2-(3-fluorobenzoyl)thieno[2,3-*b*]pyridine-5-carbonitrile (28)
- 3,6-diamino-2-(3-chlorobenzoyl)-4-(2-fluorophenyl)thieno[2,3-*b*]pyridine-5-carbonitrile (29)
- 3,6-diamino-2-(3-chlorobenzoyl)-4-(2,6-dichlorophenyl)thieno[2,3-*b*]pyridine-5-carbonitrile (30)
- 3,6-diamino-2-(3-chlorobenzoyl)-4-(2-thienyl)thieno[2,3-*b*]pyridine-5-carbonitrile (31)
- 3,6-diamino-N (4-chlorophenyl)-5-cyano-4-(2-iodophenyl)thieno[2,3-*b*]pyridine-2-carboxamide (32)
- 3,6-diamino-4-(2-bromophenyl)-*N*-(4-chlorophenyl)-5-cyanothieno[2,3-*b*]pyridine-2-carboxamide (33)
- 3,6-diamino-4-(2-chlorophenyl)-*N*-(4-chlorophenyl)-5-cyanothieno[2,3-*b*]pyridine-2-carboxamide (34)
- 3,6-diamino-4-(2-methoxyphenyl)thieno[2,3-*b*]pyridine-2,5-dicarbonitrile (35)
- 3,6-diamino-4-(2-iodophenyl)thieno[2,3-*b*]pyridine-2,5-dicarbonitrile (36)
- 3,6-diamino-4-(2-bromophenyl)thieno[2,3-*b*]pyridine-2,5-dicarbonitrile (37)
- 3,6-diamino-4-(2-chlorophenyl)thieno[2,3-*b*]pyridine-2,5-dicarbonitrile (38)
- 3,6-diamino-4-(2-ethoxyphenyl)thieno[2,3-*b*]pyridine-2,5-dicarbonitrile (39)
- 3,6-diamino-4-(2-methylphenyl)thieno[2,3-*b*]pyridine-2,5-dicarbonitrile (40)
- 3,6-diamino-4-(2-thienyl)thieno[2,3-*b*]pyridine-2,5-dicarbonitrile (41)
- 3,6-diamino-4-(1*H*-indol-3-yl)thieno[2,3-*b*]pyridine-2,5-dicarbonitrile (42)
- 3-amino-4-(2-iodophenyl)-5,6,7,8-tetrahydrothieno[2,3-*b*]quinoline-2-yl](phenyl)methanone (43)
- [3-amino-4-(2-iodophenyl)-5,6,7,8-tetrahydrothieno[2,3-*b*]quinoline-2-yl](4-chlorophenyl)methanone (44) and
- [3-amino-4-(2-bromophenyl)-5,6,7,8-tetrahydrothieno[2,3-*b*]quinoline-2-yl](phenyl)methanone (45)
- 7-amino-9-(2-chlorophenyl)-4-oxo-3,4-dihydropyrido[3',2':4,5]thieno[3,2-*d*]pyrimidine-8-carbonitrile (45)
- 7-amino-9-(2-chlorophenyl)-4-thioxo-3,4-dihydropyrido[3',2':4,5]thieno[3,2-*d*]pyrimidine-8-carbonitrile (46).

9. Compound according to anyone of the preceding claims for use as a medicament, in particular intended for the treatment of malaria.

10. A compound chosen among 3,6-diamino-2-(4-chlorobenzoyl)-4-(2-iodophenyl)thieno[2,3-*b*]pyridine-5-carbonitrile; 3,6-diamino-2-(4-chlorobenzoyl)-4-(2-chlorophenyl)thieno[2,3-*b*]pyridine-5-carbonitrile; 3,6-diamino-2-(4-chlorobenzoyl)-4-fur-2-yl)thieno[2,3-*b*]pyridine-5-carbonitrile and 3,6-diamino-2-benzoyl-4-fur-2-ylthieno[2,3-*b*]pyridine-5-carbonitrile as a medicament, in particular useful to treat malaria.

11. Compound (II), wherein Ar, A, A' and R₄ are as defined in claim 1, with the exclusion of the following four compounds of formula (II):
- (i') and (ii') wherein Ar is a phenyl group, A is a o-I or a o-Cl and A' is a hydrogen atom, R₄ represents a formula (A) wherein X is a bond, R₅ is a hydrogen atom and R₆ is a p-Cl, and
- (iii') and (iv') wherein Ar is a furyl group, A and A' are hydrogen atom, R₄ represents a formula (A) wherein X is a bond, R₅ is a hydrogen atom and R₆ is a p-Cl or a hydrogen atom, or
- Compound (VIII), wherein Ar, A, A', R₅ and R₆ are as defined in claim 1.

12. Pharmaceutical composition containing an active ingredient chosen among a compound of formula (I), (Ia), (I'a), (Ib), (I'b), (Ic) and (Id) according to anyone of claims 1 to 8 or a mixture thereof.
